# EUROPÄISCHE PATENTANMELDUNG

(11) **EP 2 745 821 A1**
(43) Veröffentlichungstag der Anmeldung: **25.06.2014**
(21) Anmeldenummer: 12198178.1
(22) Anmeldetag: 19.12.2012
(51) Int. Cl.: A61F 13/02

(54) **Haftklebendes Befestigungselement für Halbimplantate**

(71) Anmelder: LTS LOHMANN Therapie-Systeme AG, 56626 Andernach (DE)
(72) Erfinder: Hille, Thomas, 56567 Neuwied (DE)
(74) Vertreter: Michalski Hüttermann & Partner Patentanwälte

(57) **Zusammenfassung**

Die Erfindung betrifft ein haftklebendes Polymer sowie ein haftklebendes Befestigungselement für den extrakorporalen Teil eines Halbimplantats auf der Haut eines Patienten, umfassend ein haftklebendes Polymer, wobei der Gehalt an Monomeren des Polymers unter 300 ppm liegt, der Gehalt an Radikalstarter einschließlich seiner Abbauprodukte unter 100 ppm liegt, und der Gehalt an Vernetzer einschließlich seiner Abbauprodukte unter 100 ppm liegt. Es wird ein Herstellungsverfahren, die Verwendung und Halbimplantate offenbart, die das haftklebende Polymer oder Befestigungselement aufweisen.

## Beschreibung

Die vorliegende Erfindung betrifft Mittel zur Fixierung der nicht implantierbaren Teile von Halbimplantaten auf nicht intakter Haut.

Unter Halbimplantaten werden im Sinne der vorliegenden Erfindung therapeutische Vorrichtungen verstanden, die einen in den Körper eines menschlichen oder tierischen Patienten implantierbaren Teil und einen extrakorporalen Teil umfassen, wobei der implantierbare Teil und der extrakorporale Teil durch eine die Körperoberfläche des Patienten, also dessen Haut, durchdringenden Verbindung miteinander verbunden sind. Bei dem implantierbaren Teil des Halbimplantats kann es sich beispielsweise um eine Meßsonde, eine Elektrode oder eine Kanüle handeln. Der extrakorporale Teil kann zum Beispiel ein Impulsgeber oder eine Arzneistoffpumpe sein. Beispiele für Halbimplantate sind Neurostimulationssysteme, bei denen ein extrakorporaler Reizgeber über eine Drahtverbindung mit einer implantierten Elektrode verbunden ist, oder Arzneistoffpumpen, die über eine Schlauchverbindung den Wirkstoff als flüssige Zubereitung - dem Arzneistoff - in den Körper transportieren. Bevorzugte Halbimplantate zur Verabreichung von Arzneistoffen überwachen mindestens eine Körperfunktion kontinuierlich und können fehlende körpereigene Stoffe wie Hormone bedarfsgerecht substituieren.

Halbimplantate sind Medizinprodukte, die über einen längeren Zeitraum beim Patienten zur Anwendung kommen. Zudem sind Halbimplantate vergleichsweise teure Produkte zur Verabreichung von Arzneimitteln. Dementsprechend langanhaltend und zuverlässig muss eine Befestigung des extrakorporalen Teils am Patienten erfolgen. Dies gilt auch bei der Verwendung von haftklebenden Befestigungsmitteln, um den extrakorporalen Teil eines Halbimplantats unmittelbar an oder auf der Haut des Patienten zu fixieren, zumal das Gewicht des extrakorporalen Teils eines Halbimplantats vergleichsweise hoch ist und beispielswiese deutlich über dem eines transdermalen therapeutischen Systems oder einer Kanüle liegt.

Darüber hinaus sind besondere Anforderungen an den Haftkleber eines haftklebende Befestigungsmittel für den extrakorporalen Teil eines Halbimplantats zu stellen, weil die Haut des Patienten aufgrund der sie durchdringenden Verbindung des Halbimplantats nicht mehr intakt ist und somit ihre Barrierefunktion nicht mehr erfüllen kann, jedenfalls nicht im Bereich der Durchdringung, deren extrakorporaler Bereich aus Gründen der Sicherheit und Zuverlässigkeit so kurz wie möglich sein sollte. So darf ein Haftkleber für die Befestigung eines extrakorporalen Teils eines Halbimplantats auf oder an der Haut eines Patienten weder cytotoxisch noch genotoxisch sein. Das bedeutet auch, dass der Haftkleber keine cytotoxischen und/oder genotoxischen Substanzen enthalten darf. Das bedeutet, dass das haftklebende Polymer praktisch frei von extrahierbaren Verunreinigungen wie Weichmachern, Klebrigmachern, aber auch von Lösemitteln, Monomeren, Radikalstartern und/oder Vernetzern, die beispielsweise für die Herstellung des haftklebenden Polymers verwendet werden, sowie den Abbauprodukten der vorgenannten Substanzen sein muss.

Haftklebende Polymere zur Befestigung von Pflastern oder transdermalen therapeutischen Systems sind im Stand der Technik zwar bekannt, aber diese haftklebenden Polymere erfüllen nicht alle Anforderungen, die an einen Haftkleber zur Fixierung eines extrakorporalen Teils eines Halbimplantats gestellt werden.

Der vorliegenden Erfindung lag daher die Aufgabe zugrunde, ein haftklebendes Polymer für die Herstellung eines haftklebenden Befestigungselements zur Fixierung des extrakorporalen Teils eines Halbimplantats bereitzustellen, welches die vorgenannten Anforderungen erfüllt. Insbesondere sollte das haftklebende Polymer eine hohe Klebkraft haben, wasserdampfdurchlässig und hautverträglich sein. Zudem muss das haftklebende Polymer auch sterilisierbar sein. Ferner darf das haftklebende Polymer weder cytotoxisch noch genotoxisch sein.

Unter haftklebendem Polymer wird im Sinne der Erfindung ein Haftklebstoff verstanden. Ein Haftklebstoff ist ein nicht-aushärtender Klebstoff, der nach Fertigstellung der Klebstoffmasse seinen chemischen Zustand bis zum Zeitpunkt der Verklebung, während des Verklebungsvorgangs und auch während der Anwendung selber nicht verändert. Innerhalb seiner Anwendungstemperatur verfügt der Haftklebstoff über eine andauernde Oberflächenklebrigkeit (Tack), die eine Verklebung durch leichten Anpressdruck ermöglicht. Eine Zufuhr von Energie ist nicht erforderlich. So bleibt ein Haftklebstoff dauerklebrig und kann nicht nur wieder abgelöst werden, sondern ist auch wiederverklebbar. Typisch für einen Haftklebstoff ist ein dynamischer Adhäsionsaufbau, der bei unterschiedlichen Haftklebstoffen unterschiedlich lange dauern und zu unterschiedlich hohen Endfestigkeiten führen kann.

Haftklebende Polymere gewährleisten einen ausreichenden Halt auf der Haut eines Patienten nur dann, wenn sie klebrig machende Harze enthalten, beispielsweise Kolophonium oder eines seiner Derivate. Bei den klebrig machenden Harzen handelt es sich jedoch um Naturprodukte, die sich hinsichtlich ihrer quantitativen und qualitativen Zusammensetzung von Charge zu Charge unterscheiden. Dies ist für eine Anwendung dieser klebrig machenden Harze als Kleber auf intakter Haut zwar gerade noch akzeptabel, für eine Applikation auf offener Haut jedoch nicht.

Wundschnellverbände, die so genannten Heftpflaster, werden zwar auf offener Haut appliziert, wiegen aber deutlich weniger als die nicht implantierten Teile eines Halbimplantats, und sind auch deutlich kleiner. Daher muss die Klebkraft eines Haftklebers zur Befestigung eines Wundschnellverbandes nicht so hoch sein, wie die Klebkraft eines haftklebenden Polymers für das Fixieren des extrakorporalen Teils eines Halbimplantats. Zudem stellt es keinen nennenswerten wirtschaftlichen Schaden dar, wenn ein Heftpflaster nur wenige Stunden auf der Haut haftet und dann durch ein neues Heftpflaster ersetzt werden muss. Dagegen ist der wirtschaftliche Schaden erheblich größer, wenn der extrakorporale Teil eines in Rede stehenden Halbimplantats verloren geht oder kaputt geht, weil er nicht zuverlässig auf der Haut des Patienten haftet.

Um eine lange Tragdauer zu gewährleisten, muss eine haftklebende Polymerschicht wasserdampfdurchlässig sein. Haftklebende Polymere aus der Gruppe der synthetischen Kautschuke, beispielsweise Polyisobutylen, weisen zwar eine hohe Klebkraft auf, sind aber nicht wasserdampfdurchlässig.

Wässrige Dispersionskleber könnten zwar eine ausreichende Klebkraft haben, kommen aber wegen ihrer Keimbelastung nicht in Frage.

Acrylathaftkleber sind wasserdampfdurchlässig. Besonders gut auf Haut klebene Polyacrylate sind solche, die unter Verwendung freier Acrylsäure hergestellt wurden. Für das Haftkleben auf Haut besonders gut geeignete Polyacrylate weisen eine Säurezahl zwischen 0,5 und 7,5 auf. Bei Polyacrylaten, die eine niedrigere Säurezahl aufweisen, ist die Haftung auf Haut zu niedrig. Allerdings darf das Polyacrylat auch keinen zu hohen Anteil an Acrylsäure aufweisen, da ein direkter Zusammenhang zwischen dem Anteil an in der Polymerkette gebundener Acrylsäure und Hautreizung besteht.

Zudem weisen Acrylathaftkleber, wie sie bereits für die Herstellung von therapeutischen Systemen zur transdermalen Verabreichung von Wirkstoffen über intakte Haut verwendet werden, einen hohen Anteil an nicht abreagierten Monomeren auf, der im einstelligen Prozentbereich liegt. Dem Fachmann ist auch bekannt, dass Acrylate aufgrund ihrer alpha, beta ungesättigten Carbonylstruktur Gegenstand der Michael-Addition sein können. Damit sind sie potentiell genotoxisch.

Der vorliegenden Erfindung lag somit die Aufgabe zugrunde, einen Haftkleber für ein haftklebendes Befestigungselement bereitzustellen, der eine dauerhafte, also bis zu 7 Tage andauernden, sichere Befestigung des extrakorporalen Teils eines Halbimplantats bereitzustellen, wobei der Haftkleber weder Weichmacher noch Klebrigmacher enthalten darf, weder cytotoxisch noch genotoxisch sein dart, jedoch wasserdampfdurchlässig und auch sterilisierbar sein muss.

Die Aufgabe wird überraschenderweise durch ein Verfahren zur Herstellung einer haftklebenden Polymerschicht gelöst, bei dem die Trocknung der Polymerschicht unter Bedingungen erfolgt, bei denen der Gehalt an Monomeren des Polymers auf unter 300 ppm, der Gehalt an Radikalstarter und/oder seiner Abbauprodukte unter 100 ppm und der Gehalt an Vernetzer und/oder seiner Abbauprodukte unter 100 ppm gesenkt wird.

In einem ersten Aspekt betrifft die Erfindung ein haftklebendes Befestigungselement für den extrakorporalen Teil eines Halbimplantats an der Haut eines Patienten, wobei das Befestigungselement ein haftklebendes Polymer umfasst, das die vorgenannten Anforderungen erfüllt.

In einem zweiten Aspekt betrifft die Erfindung ein Verfahren zur Herstellung eines haftklebenden Befestigungselements gemäß dem ersten Aspekt.

In einem dritten Aspekt betrifft die Erfindung die Verwendung des haftklebenden Befestigungselements zur Fixierung des extrakorporalen Teils eines Halbimplantats an oder auf der Haut eines Patienten.

In einem weiteren Aspekt betrifft die Erfindung ein Halbimplantat, deren extrakorporaler Teil das haftklebende Befestigungselement gemäß dem ersten Aspekt umfasst.

In einem weiteren Aspekt betrifft die Erfindung ein haftklebendes Polymer, das die vorgenannten Anforderungen für die Befestigung eines medizinischen Gegenstands auf nicht intakter Haut erfüllt.

Gemäß dem ersten Aspekt betrifft die Erfindung ein haftklebendes Befestigungselement für den extrakorporalen Teil eines Halbimplantats an der Haut eines Patienten, umfassend ein haftklebendes Polymer, bei dem der Gehalt an Monomeren des Polymers unter 300 ppm liegt, der Gehalt an Radikalstarter und/oder seinen Abbauprodukten unter 100 ppm liegt, und der Gehalt an Vernetzer und/oder seinen Abbauprodukten unter 100 ppm liegt.

Im Sinne der vorliegenden Erfindung umfasst der Begriff "haftklebendes Polymer" auch Mischungen von zwei oder mehr Polymeren.

Bei einer Ausführungsform des haftklebenden Befestigungselements liegt das haftklebende Polymer in Form einer Schicht vor, also in Form einer haftklebenden Polymerschicht.

In einer zusätzlichen Ausführungsform weist die haftklebende Polymerschicht ein Flächengewicht von etwa 90 g/m² oder mehr auf, vorzugsweise von etwa 100 g/m² auf. Bei einem geringeren Flächengewicht reicht die Klebkraft der haftklebenden Polymerschicht auf der Haut nicht aus, um den extrakorporalen Teil eines Halbimplantats sicher zu fixieren. Ein Flächengewicht von etwa 100 g/m² führt zu einer Schichtdicke von etwa 100 µm.

In einer anderen und/oder zusätzlichen Ausführungsform weist die haftklebende Polymerschicht ein Flächengewicht von etwa 110 g/m² auf. In noch einer anderen und/oder zusätzlichen Ausführungsform weist die haftklebende Polymerschicht ein Flächengewicht auf, das kleiner oder gleich 125 g/m² ist. Ein höheres Flächengewicht ist nicht nur wirtschaftlich unsinnig, sondern erschwert auch das Entfernen der unerwünschten Verunreinigungen beim Trocknen.

In einer anderen und/oder zusätzlichen Ausführungsform ist das haftklebende Polymer ein Vinylacetat-Acrylat-Copolymer. Ein Vorteil bei der Verwendung von Polyacrylaten wie dem Vinylacetat-Acrylat-Copolymer liegt darin, dass sie durch Lösungspolymerisation in einem organischen Lösungsmittel oder Lösungsmittelgemisch hergestellt werden. In einem organischen Lösungsmittel oder Lösungsmittelgemisch können Mikroorganismen jedoch nicht wachsen. Daher ist eine mikrobielle Kontamination im Rahmen der Herstellung von Polyacrylaten nicht zu befürchten.

In einer zusätzlichen Ausführungsform weist das Vinylacetat-Acrylat-Copolymer einen Anteil von mindestens 0,5 Gew.-%, vorzugsweise von mindestens 5 Gew.-% Acrylsäure auf, der in der Polymerkette gebunden ist. Ein solcher Anteil an Acrylsäure führt zu einem Vinylacetat-Acrylat-Copolymer mit einer Säurezahl von 10 - 50, bezogen auf den getrockneten Polymer. Die Säurezahl gibt an, wieviel Milligramm KOH von 1 g Polymer zur Neutralisation benötigt wird. Zwar ist die Säurezahl von Polymeren nicht so einfach zu bestimmen wie die niedermolekularer Verbindungen, aber die Bestimmung der Säurezahl eines pharmazeutisch genutzten Polymers ist ein im Europäischen Arzneibuch (Ph. Eur.) beschriebenes Standardverfahren.

Dementsprechend kann auch die Säurezahl eines Polymers mit dem Fachmann bekannten Standardverfahren bestimmt werden. In einer anderen oder zusätzlichen Ausführungsform ist die Säurezahl des Vinylacetat-Acrylat-Copolymers nicht höher als 75, vorzugsweise nicht höher als 50. Der Anteil an Acrylsäure , dem in der Polymerkette des Vinylacetat-Acrylat-Copolymers gebunden ist, beträgt in einer weiteren und/oder zusätzlichen Ausführungsform nicht mehr als 7,5 Gew.-%.

In einer weiteren und/oder alternativen Ausführungsform weist das haftklebende Befestigungselement eine Trägerschicht auf, die zumindest einseitig mit dem haftklebenden Polymer beschichtet ist. In einer anderen Ausführungsform ist die Trägerschicht beidseitig mit dem haftklebenden Polymer beschichtet. Das bedeutet, dass die Trägerschicht auf einer ihrer Flächen oder auf beiden Flächen eine haftklebende Polymerschicht aufweist.

Sofern das Befestigungselement eine Trägerschicht oder Rückschicht umfasst, auf der die haftklebende Polymerschicht aufgebracht ist, muss auch diese Träger- oder Rückschicht wasserdampfdurchlässig sein, um eine lange Tragdauer des auf der Haut fixierten Gegenstands zu gewährleisten.

Eine Trägerschicht ist wasserdampfdurchlässig im Sinne der Erfindung, wenn bei der Bestimmung der Wasserdampfdurchlässigkeit messbare Werte gefunden werden. Bei der Bestimmung der Wasserdampfdurchlässigkeit wiegt man Wasser in eine Petrischale ein, deckt diese mit der zu prüfenden Trägerschicht hermetisch ab und stellt die Petrischale (Durchmesser: 100 mm) für 24 Stunden in einen Trockenschrank bei Raumtemperatur. Anschließend bestimmt man gravimetrisch den Wasserverlust und errechnet die Wasserdampfdurchlässigkeit aus der Differenz, die in der Einheit [g/m² x 24 Stunden] angegeben wird. Eine Trägerschicht wird im Sinne der vorliegenden Offenbarung als wasserdampfdurchlässig angesehen, wenn ihre Wasserdampfdurchlässigkeit mit ≥ 10 g/m² x 24 h bestimmt wurde.

Grundsätzlich kämen Polyurethanfolien zwar als Rückschicht in Frage, sie sind jedoch nicht sterilisierbar. Das für die Sterilisation von Medizinprodukten üblicherweise verwendete Verfahren der Bestrahlung des Gegenstands mit Gamma-Strahlung führt zu einer deutlichen Gelbfärbung des Polyurethans. Polyester, beispielsweise Polyethylenterephthalat, sind zwar sterilisierbar, aber in Form von Folien nicht wasserdampfdurchlässig.

In einer Ausführungsform ist die Trägerschicht aus der Gruppe ausgewählt, die Gewebe, Gewirke, Gelege, Vliese, wasserdampfdurchlässige Folien und perforierte Folien umfasst. In einer weiteren Ausführungsform ist das Gewebe, Gewirke, Gelege oder Vlies behandelt, um beispielsweise die Anforderungen an Festigkeit zu erfüllen, beispielsweise durch Vernadeln, Verdichten, Verkleben oder andere, dem Fachmann bekannte chemische und/oder physikalische Verfahren. In alternativen Ausführungsformen ist die Trägerschicht eine wasserdampfdurchlässige Folie. Das bedeutet, dass die Folie an sich aufgrund des Materials aus dem sie besteht wasserdampfdurchlässig ist. Die Folie kann aber auch aus einem nicht wasserdampfdurchlässigen Material bestehen und weist dann eine perforierte Fläche auf, durch die Wasserdampf durchtreten kann.

In einer Ausführungsform ist die Trägerschicht ein Polymergewebe. Dadurch, dass die Trägerschicht nicht in Form einer Folie, sondern als Gewebe vorliegt, wird die Wasserdampfdurchlässigkeit der Trägerschicht gewährleistet. Ein Vorteil liegt darin, dass die Trägerschicht aus einem Polymer oder Polymergemisch bestehen kann, welches als Folie vorliegend wasserdampfundurchlässig wäre.

In einer anderen und/oder zusätzlichen Ausführungsform besteht das Polymergewebe aus Polyester. Gemäß einer weiteren Ausführungsform ist der Polyester Polyethylenterephthalat.

Die vorliegende Erfindung erstreckt sich auch auf Halbimplantate, die einen extrakorporalen Teil umfassen, der ein haftklebendes Befestigungselement wie vorstehend beschrieben aufweist.

In einer Ausführungsform weist der extrakorporale Teil des Halbimplantats das haftklebende Befestigungselement nicht in Form eines zumindest einseitig mit der haftklebenden Polymerschicht versehenen Polymergewebes vor, sondern in Form einer haftklebenden Polymerschicht, die ohne zusätzliche Trägerschicht auf dem Bereich des extrakorporalen Teils des Halbimplantats angeordnet ist, mit dem der extrakorporale Teil auf der Haut des Patienten befestigt werden soll.

Das erfindungsgemäße Befestigungselement ermöglicht die sichere Befestigung des extrakorporalen Teils eines Halbimplantats direkt auf der Haut eines Patienten über einen Zeitraum von mindestens einem Tag, vorzugsweise über einen Zeitraum von bis zu 7 Tagen. Darüber hinaus wird durch das erfindungsgemäße Befestigungselement auch bei Ausübung von Sport, selbst bei Ausübung von Hochleistungssport, eine sichere Befestigung des extrakorporalen Teils eines Halbimplantats direkt auf der Haut über den genannten Zeitraum hinweg ermöglicht.

Gemäß dem zweiten Aspekt betrifft die Erfindung ein Verfahren zur Herstellung eines haftklebenden Befestigungselements gemäß dem ersten Aspekt. Bei dem Verfahren durchläuft das haftklebende Polymer nach erfolgter Polymerisation und Vernetzung mindestens einem Trocknungsschritt. Dieser Trocknungsschritt wird bei einer Temperatur durchgeführt, bei der in der Lösung oder Dispersion des haftklebenden Polymers enthaltene Reste an nicht abreagieren Monomeren, Radikalstartern, Vernetzern und/oder deren Abbauprodukten so weit entfernt werden, dass der der Gehalt an Monomeren des Polymers unter 300 ppm liegt, der Gehalt an Radikalstarter und/oder seiner Abbauprodukte unter 100 ppm liegt, und der Gehalt an Vernetzer und/oder seiner Abbauprodukte unter 100 ppm liegt. Diese Temperatur ist höher als die Temperatur, mit der das haftklebende Polymer bei Verwendung als Haftkleber für Wundschnellverbände oder transdermale therapeutische Systeme üblicherweise getrocknet wird. Sofern es sich bei dem haftklebenden Polymer um ein Vinylacetat-Acrylat-Copolymer handelt, insbesondere um eines der zuvor näher beschriebenen Vinylacetat-Acrylat-Copolymere, erfolgt die Trocknung bei einer Ausführungsform des erfindungsgemäßen Verfahrens bei einer um etwa 35°C höheren Temperatur als üblicherweise. Dazu kann von der Maßnahme Gebrauch gemacht werden, dass anstelle des üblicherweise verwendeten Prozessliners silikonisiertes Papier ein anderer Prozessliner verwendet wird, der einer höhere Trocknungstemperatur ermöglicht. In einer Ausführungsform erfolgt die Trocknung bei einer Temperatur von etwa 115°C.

In einer weiteren Ausführungsform erfolgt die Trocknung nachdem ein Träger mit der das haftklebende Polymer enthaltenden Lösung oder Dispersion beschichtet wurde.

In einer anderen und/oder weiteren Ausführungsform ist der Träger die Trägerschicht des haftklebenden Befestigungselements.

Gemäß dem dritten Aspekt betrifft die Erfindung die Verwendung des haftklebenden Befestigungselements zur Fixierung des extrakorporalen Teils eines Halbimplantats an oder auf der Haut eines Patienten. Da das erfindungsgemäße Befestigungselement nicht nur eine dauerhafte und zuverlässige Befestigung eines medizinischen Gegenstands an der Haut eines Patienten ermöglicht, sondern auch weder cytotoxisch noch genotoxisch ist, ist es für die Fixierung des extrakorporalen Teils eines Halbimplantats auf nicht intakter Haut geeignet, insbesondere in dem Bereich, indem die Haut durchdrungen wird, beispielsweise von der Verbindung zwischen dem implantierten Teil und dem extrakorporalen Teil eines Halbimplantats.

In einer Ausführungsform der Verwendung kann der extrakorporale Teil des Halbimplantats auf der Haut des Patienten befestigt werden, indem das haftklebende Befestigungselement als Klebeband über den extrakorporalen Teil gelegt und mit seinen überstehenden Enden auf die Haut geklebt wird. In einer anderen oder zusätzlichen Ausführungsform der Verwendung kann das haftklebende Befestigungselement als doppelseitiges Klebeband verwendet werden, um mit einer seiner beiden Flächen auf der hautseitigen Fläche des extrakorporalen Teils des Halbimplantats und mit seiner anderen Fläche auf der Haut des Patienten geklebt zu werden. Die vorgenannten Ausführungsformen umfassen auch die Verwendungen, bei denen der extrakorporale Teil nicht direkt, sondern über einen gesonderten, in den der extrakorporale Teil des Halbimplantats formschlüssig eingepasst und beispielsweise eingerastet oder anderweitig festgehalten werden kann, auf der Haut fixiert wird. Insofern sind im Sinne der Erfindung auch derartige Halter als zu dem extrakorporalen Teil eines Halbimplantats gehörend anzusehen.

Gemäß einem weiteren Aspekt betrifft die Erfindung ein haftklebendes Polymer, das die vorgenannten Anforderungen für die Befestigung eines medizinischen Gegenstands auf nicht intakter Haut erfüllt, also die Befestigung eines medizinischen Gegenstands auf nicht intakter Haut geeignet ist. Dieses haftklebende Polymer zeichnet sich durch einen Gehalt an Monomeren des Polymers aus, der unter 300 ppm liegt, einen Gehalt an Radikalstarter und/oder seiner Abbauprodukte, der unter 100 ppm liegt, und einen Gehalt an Vernetzer und/oder seiner Abbauprodukte, der unter 100 ppm liegt.

In einer anderen und/oder zusätzlichen Ausführungsform ist das haftklebende Polymer ein Vinylacetat-Acrylat-Copolymer. Ein Vorteil bei der Verwendung von Polyacrylaten wie dem Vinylacetat-Acrylat-Copolymer liegt darin, dass sie durch Lösungspolymerisation in einem organischen Lösungsmittel oder Lösungsmittelgemisch hergestellt werden. In einem organischen Lösungsmittel oder Lösungsmittelgemisch können Mikroorganismen jedoch nicht wachsen. Daher ist eine mikrobielle Kontamination im Rahmen der Herstellung von Polyacrylaten nicht zu befürchten.

In einer zusätzlichen Ausführungsform hat das Vinylacetat-Acrylat-Copolymer eine Säurezahl von mindestens 0,5, vorzugsweise von mindestens 5. In einer anderen oder zusätzlichen Ausführungsform ist die Säurezahl des Vinylacetat-Acrylat-Copolymers nicht höher als 75, vorzugsweise nicht höher als 50.

In einer anderen und/oder zusätzlichen Ausführungsform weist das Vinylacetat-Acrylat-Copolymer einen Anteil von mindestens 0,5 Gew.-% Acylsäure auf, vorzugsweise einen Anteil von mindestens 5 Gew.-% Acrylsäure. In einer weiteren Ausführungsform beträgt der Anteil an Acrylsäure in dem Vinylacetat-Acrylat-Copolymer nicht mehr als 7,5 Gew.-%. Die vorgenannten Anteile an Acrylsäure im Vinylacetat-Acrylat-Copolymer beziehen sich auf den in der Polymerkette gebundenen Anteil an Acrylsäure.

Die vorliegende Erfindung wird nachfolgend anhand von Ausführungsbeispielen veranschaulicht, wobei die Ausführungsbeispiele nur zur Erläuterung dienen, aber die vorliegende Erfindung nicht einschränken. Die vorliegende Erfindung wird ausschließlich durch die Ansprüche definiert.

### Beispiele

### Beispiel 1 - Herstellung eines haftklebenden Polymers

Für die Herstellung eines haftklebenden Polymers wurden die Monomere Vinylacetat, Acrylsäure, Butylacrylat und 2-Ethylhexylacrylat in einem Gewichtsverhältnis von 5:5:15:75 gemischt und in Ethylacetat gelöst. Die resultierende Mischung der Monomere wurde mit Azoisobutyronitril (AIBN) zur Polymerisation gebracht. Nach beendeter Polymerisation wurde das Vinylacetat-Acrylat-Copolymerisat so mit Heptan verdünnt, dass eine 50 Gew.-%ige Lösung des Polymers im Lösungsmittelgemisch Ethylacetat/Heptan entstand. Eine entsprechende Polymerlösung ist kommerziell erhältlich.

In der Lösung des Vinylacetat-Acrylat-Copolymerisats im Lösungsmittelgemisch Ethylacetat/Heptan konnten Restmengen an den Monomeren Vinylacetat, Acrylsäure, Butylacrylat und 2-Ethylhexylacrylat im Größenbereich von 1 Gew.-% nachgewiesen werden. Zusätzlich wurde bei den Analysen des Vinylacetat-Acrylat-Copolymerisats auch Tetramethylsuccinodinitril (TMSN), ein Zersetzungsprodukt des AIBN, in der Polymerlösung nachgewiesen. Für die Nachweise wurden gebräuchliche, dem Fachmann bekannte gaschromatographische Verfahren verwendet.

Vor der weiteren Verwendung des Vinylacetat-Acrylat-Coplomers wurde so viel einer Suspension von Aluminiumacetylacetonat (Vernetzer) in Ethanol zu der Lösung des Vinylacetat-Acrylat-Copolymerisats in Ethylacetat/Heptan zugegeben, dass Aluminium und Polymer in einem Gewichtsverhältnis von 0,2 : 99,8 vorlagen. Mit der so erhaltenen Mischung erfolgten die in den Beispielen 2 und 3 beschriebenen Beschichtungen.

### Beispiel 2 - Vergleichsbeschichtung

Silikonisiertes Papier wurde mit 630 kg der in Beispiel 1 beschriebenen Lösung aus Aluminiumacetylacetonat versetztem Vinylacetat-Acrylat-Copolymerisats auf einer industriellen Beschichtungsanlage beschichtet. Die Beschichtung wurde in einer Trockenanlage bei einer üblichen Trockentemperatur von 80 °C getrocknet. Es wurde eine Beschichtung mit einem Flächengewicht von etwa 100 g/m² erhalten. Anschließend wurde die haftklebende Fläche des Laminats aus silikonisiertem Papier und haftklebender Vinylacetat-Acrylat-Copolymerschicht mit einem Polyethylenterephthalat-Gewebe (PET-Gewebe) abgedeckt.

Nach dem Entfernen des Papiers wurde die Wasserdampfdurchlässigkeit des Laminats aus Vinylacetat-Acrylat-Copolymerschicht und PET-Gewebe bestimmt. Die Wasserdampfdurchlässigkeit lag hier bei 150 g/m² x 24 h.

**Tabelle 1: Analyseergebnisse einer bei 80 °C getrockneten Haftkleberschicht auf Basis eines Vinylacetat-Acrylat-Copolymers.**

| | | Analytisches Ergebnis | Spezifikation gem. Ph. Eur. |
|---|---|---|---|
| Lösemittel | | | |
| | Ethanol | unterhalb der Nachweisgrenze | max. 0,5 % |
| | Heptan | unterhalb der Nachweisgrenze | max. 0,5 % |
| | Ethylacetat | unterhalb der Nachweisgrenze | max. 0,5 % |
| Monomere | | | |
| | Acrylsäure | unterhalb der Nachweisgrenze | |
| | Butylacrylat | unterhalb der Nachweisgrenze | |
| | Vinylacetat | unterhalb der Nachweisgrenze | |
| | 2-Ethylhexylacrylat | ≥ 0,15 % | |
| Radikalstarter | | | |
| | TMSN | ≥ 300 ppm | |
| Vernetzer | | | |
| | Acetylacetonat | unterhalb der Nachweisgrenze | |

Routinemäßig durchgeführte gaschromatographische Untersuchungen auf niedermolekulare Verunreinigungen der haftklebenden Vinylacetat-Acrylat-Copolymerschicht, die aus dem Herstellungsprozess resultieren, ergaben die in der Tabelle 1 zusammengefassten Ergebnisse.

Aufgrund der vorstehenden Analyseergebnisse erfüllt die haftklebende Polymerschicht die im Europäischen Arzneibuch (Pharmacopoea Europaea, Ph. Eur.) festgelegten Reinheitsanforderungen für haftklebende Bandagen. Daher könnte das mit dem Vinylacetat-Acrylat-Copolymer beschichtete und PET-Gewebe zur Herstellung Bandage für die Fixierung von Wundschnellverbänden oder transdermalen therapeutischen Systemen auf intakter menschlicher Haut verwendet werden. Allerdings sind der Gehalt an TMSN und der Gehalt an 2-Ethylhexylacrylat in der haftklebenden Polymerschicht zu hoch, um diese bei nicht intakter Haut zur Fixierung eines Gegenstandes zu verwenden.

### Beispiel 3 - Ausführungsbeispiel

Eine silikonisierte PET-Folie wurde auf einer industriellen Beschichtungsanlage mit 630 kg der in Beispiel 1 beschriebenen Lösung aus Aluminiumacetylacetonat versetztem Vinylacetat-Acrylat-Copolymerisat beschichtet. Die mit dem Vinylacetat-Acrylat-Copolymerisat beschichtete PET-Folie wurde in einer Trocknungsanlage bei 115 °C getrocknet. Es wurde eine Polymerschicht mit einem Flächengewicht von 100 g/m² erhalten. Anschließend wurde die haftklebende Fläche des Laminats aus silikonisierter PET-Folie und haftklebender Vinylacetat-Acrylat-Copolymerschicht mit einem Polyethylenterephthalat-Gewebe (PET-Gewebe) abgedeckt.

Die gaschromatographischen Untersuchungen auf niedermolekulare Verunreinigungen der haftklebenden Vinylacetat-Acrylat-Copolymerschicht, die aus dem Herstellungsprozess resultieren, ergaben die in der Tabelle 2 zusammengefassten Ergebnisse.

**Tabelle 2: Analyseergebnisse einer bei 115 °C getrockneten Haftkleberschicht auf Basis eines Vinylacetat-Acrylat-Copolymers.**

| | | Analytisches Ergebnis | Spezifikation gem. Ph. Eur. |
|---|---|---|---|
| Lösemittel | | | |
| | Ethanol | unterhalb der Nachweisgrenze | max. 0,5 % |
| | Heptan | unterhalb der Nachweisgrenze | max. 0,5 % |
| | Ethylacetat | unterhalb der Nachweisgrenze | max. 0,5 % |
| Monomere | | | |
| | Acrylsäure | unterhalb der Nachweisgrenze | |
| | Butylacrylat | unterhalb der Nachweisgrenze | |
| | Vinylacetat | unterhalb der Nachweisgrenze | |
| | 2-Ethylhexylacrylat | < 300 ppm | |
| Radikalstarter | | | |
| | TMSN | < 100 ppm | |
| Vernetzer | | | |
| | Acetylacetonat | unterhalb der Nachweisgrenze | |

Aufgrund dieser Analyseergebnisse dürfte das Vinylacetat-Acrylat-Copolymer als Haftkleber nicht cytotoxisch sein und für einen direkten Kontakt mit nicht intakter Haut geeignet sein.

### Beispiel 4 - Cytotoxizitätstest

Um festzustellen, ob die gemäß Beispiel 2 hergestellte Haftkleberschicht für einen direkten Kontakt mit nicht intakter Haut geeignet ist, wurden Cytotoxizitätstests gemäß DIN EN ISO 10993-5 sowohl im direkten als auch im indirekten Kontakt durchgeführt.

In den Cytotoxizitätstests zeigte sich die gemäß Beispiel 2 hergestellte Haftkleberschicht als nicht zytotoxisch. Daher ist ein gemäß Beispiel 2 hergestellter Haftkleber geeignet, um einen Gegenstand wie den extrakorporalen Teil eines Halbimplantats unter direktem Kontakt mit nicht intakter Haut auf dieser zu befestigen.

### Beispiel 5 - Klebkraftbestimmung

Die Klebkraft des gemäß Beispiel 2 hergestellten Haftkleber wurde nach einer Methode in Anlehnung an die AFERA Norm bestimmt. Zu diesem Zweck wurden aus dem Laminat aus silikonisierter PET-Folie und haftklebender Vinylacetat-Acrylat-Copolymerschicht quadratische Stanzlinge mit einer Kantenlänge von 72 mm hergestellt. Deren Klebkraft wurde an einer Stahlplatte und einem Abzugswinkel von 90° mit etwa 58 N/Stanzling bestimmt. Hierbei ist allerdings zu berücksichtigen, dass einerseits eine gemäß AFERA Norm mit Hilfe einer Stahlplatte bestimmte Klebkraft nicht zwingend auf Haut als Klebsubstrat übertragbar ist, und es andererseits für Haut als Klebsubstrat keinen normierten Klebkrafttest gibt.

Durch ihre außerordentlich hohe Klebkraft - auch auf Haut - ist die gemäß Beispiel 2 hergestellte Haftkleberschicht geeignet, einen Gegenstand wie den extrakorporalen Teil eines Halbimplantats unter direktem Kontakt auf Haut langanhaltend zu befestigen.

## Patentansprüche

1. Ein haftklebendes Befestigungselement für den extrakorporalen Teil eines Halbimplantats auf der Haut eines Patienten, umfassend ein haftklebendes Polymer, **dadurch gekennzeichnet, dass** der Gehalt an Monomeren des Polymers unter 300 ppm liegt, der Gehalt an Radikalstarter und/oder seiner Abbauprodukte unter 100 ppm liegt, und der Gehalt an Vernetzer und/oder seiner Abbauprodukte unter 100 ppm liegt.

2. Das haftklebende Befestigungselement gemäß Anspruch 1, **dadurch gekennzeichnet, dass** das haftklebende Polymer in Form einer Schicht vorliegt.

3. Das haftklebende Befestigungselement gemäß Anspruch 2, **dadurch gekennzeichnet, dass** die haftklebende Polymerschicht ein Flächengewicht aufweist, das kleiner oder gleich 125 g/m² ist, vorzugsweise ein Flächengewicht von etwa 90 g/m², besonders bevorzugt ein Flächengewicht von etwa100 g/m² und ganz besonders bevorzugt ein Flächengewicht von 110 g/m² aufweist.

4. Das haftklebende Befestigungselement gemäß einem der vorhergehenden Ansprüche, **dadurch gekennzeichnet, dass** das haftklebende Polymer ein Vinylacetat-Acrylat-Copolymer ist.

5. Das haftklebende Befestigungselement gemäß Anspruch 4, **dadurch gekennzeichnet, dass** das Vinylacetat-Acrylat-Copolymer eine Säurezahl von mindestens 0,5 hat, vorzugsweise von mindestens 5, und die Säurezahl nicht höher als 75, vorzugsweise nicht höher als 50 ist.

6. Das haftklebende Befestigungselement gemäß Anspruch 4 oder 5, **dadurch gekennzeichnet, dass** das Vinylacetat-Acrylat-Copolymer einen Anteil von mindestens 0,5 Gew.-% Acrylsäure aufweist, vorzugsweise einen Anteil von mindestens 5 Gew.-% Acrylsäure, und dass der Anteil an Acrylsäure in dem Vinylacetat-Acrylat-Copolymer nicht mehr als 7,5 Gew.-% beträgt.

7. Das haftklebende Befestigungselement gemäß einem der vorangehenden Ansprüche, **dadurch gekennzeichnet, dass** es eine Trägerschicht aufweist, die zumindest einseitig mit dem haftklebenden Polymer beschichtet ist.

8. Das haftklebende Befestigungselement gemäß Anspruch 7, **dadurch gekennzeichnet, dass** die Trägerschicht wasserdampfdurchlässig ist.

9. Das haftklebende Befestigungselement gemäß Anspruch 7 oder 8, **dadurch gekennzeichnet, dass** die Trägerschicht aus der Gruppe ausgewählt ist, die Gewebe, Gewirke, Gelege, Vliese, wasserdampfdurchlässige Folien und perforierte Folien umfasst.

10. Das haftklebende Befestigungselement gemäß Anspruch 9, **dadurch gekennzeichnet, dass** das Gewebe ein Polymergewebe ist, das vorzugsweise aus einem Polyester besteht, besonders bevorzugt aus Polyethylenterephthalat.

11. Ein Verfahren zur Herstellung eines haftklebenden Befestigungselements gemäß einem der Ansprüche 1 bis 10, **dadurch gekennzeichnet, dass** das haftklebende Polymer nach erfolgter Polymerisation und Vernetzung mindestens einem Trocknungsschritt unterzogen wird, der bei einer Temperatur durchgeführt wird, bei der in der Lösung oder Dispersion des haftklebenden Polymers enthaltene Reste an nicht abreagieren Monomeren, Radikalstartern, Vernetzern und ihrer Abbauprodukte so weit entfernt werden, dass der der Gehalt an Monomeren des Polymers unter 300 ppm liegt, der Gehalt an Radikalstarter und/oder seiner Abbauprodukte unter 100 ppm liegt, und der Gehalt an Vernetzer und/oder seiner Abbauprodukte unter 100 ppm liegt.

12. Das Verfahren gemäß Anspruch 11, **dadurch gekennzeichnet, dass** das haftklebende Polymer ein Vinylacetat-Acrylat-Copolymer gemäß Anspruch 4 oder 5 ist, und die Trocknung bei einer um etwa 35°C höheren Temperatur als üblicherweise erfolgt, vorzugsweise bei einer Temperatur von etwa 115 °C.

13. Verwendung eines haftklebenden Befestigungselements gemäß einem der Ansprüche 1 bis 10 zur Befestigung des extrakorporalen Teils eines Halbimplantats auf der Haut eines Patienten.

14. Ein Halbimplantat, umfassend einen extrakorporalen Teil, **dadurch gekennzeichnet, dass** der extrakorporale Teil ein haftklebendes Befestigungselement gemäß einem der Ansprüche 1 bis 10 aufweist.

15. Ein haftklebendes Polymer für die Befestigung des extrakorporalen Teils eines Halbimplantats auf nicht intakter Haut, **dadurch gekennzeichnet, dass** der Gehalt an Monomeren des Polymers unter 300 ppm liegt, der Gehalt an Radikalstarter und/oder seiner Abbauprodukte unter 100 ppm liegt, und der Gehalt an Vernetzer und/oder seiner Abbauprodukte unter 100 ppm liegt.
